# EUROPEAN PATENT SPECIFICATION

(11) **EP 3 154 429 B1**
(45) Date of publication and mention of the grant of the patent: **30.12.2020**
(21) Application number: 15805748.9
(22) Date of filing: 11.06.2015
(51) Int. Cl.: A61B 5/15, B01L 3/00, G01N 1/02, G01N 1/38, B01L 3/02, G01N 1/28

(54) **SAMPLE DELIVERY SYSTEM**
PROBENAUSGABESYSTEM
SYSTÈME DE DISTRIBUTION D'ÉCHANTILLON

(30) Priority: 13.06.2014 US 201462011609 P
(43) Date of publication of application: 19.04.2017
(60) Divisional application: 18196227.5 / 3 505 067
(73) Proprietor: Siemens Healthcare Diagnostics Inc., Tarrytown, NY 10591 (US)
(72) Inventor: LEDDEN, David J., Medway, Massachusetts 02053 (US); COX, Janine A., Stoughton, Massachusetts 02072 (US)
(74) Representative: Schweitzer, Klaus
(86) International application number: PCT/US2015/035326
(87) International publication number: WO 2015/191853

(56) References cited:
- US-A- 3 475 127
- US-A- 3 630 191
- US-A- 4 589 421
- US-A- 4 866 097
- US-A- 5 257 984
- US-A- 6 102 896
- US-A1- 2002 082 522
- US-A1- 2008 081 378
- US-A1- 2014 017 147
- US-B1- 6 368 356

## Description

### BACKGROUND

### 1. Field of the Disclosure

This disclosure relates to a device for faster sample delivery into a liquid medical assay.

### 2. Brief Description of the Related Art

In certain liquid based medical assay devices, a portion of a sample liquid to be tested is retained in a capillary tube. The capillary tube is then fully or partially submerged into a second liquid, such as an assay diluent (also known as an assay reaction solution). The portion of the sample liquid in the capillary tube then diffuses into the assay reaction solution. The diffusion of the portion of the sample liquid out of the capillary tube, however, is a relatively slow process.

Sample delivery systems are known in the prior art.

US 4,866,097 pertains to a device for the controlled release of an active material into an aqueous medium which comprises a water soluble capillary tube the bore of which contains an active material.

US 2014/0017147 A1 relates to an integrated sampling and dispensing device comprising a flexible container, a dropper with a sampling rod and a sample removing vessel disposed therebetween which can be conveniently used for collecting specimen and dispensing the same after a reaction in a single device by severing the sampling rod and turning the container upside down.

### SUMMARY OF THE CONCEPTS OF THE DISCLOSURE

In one aspect, herein disclosed examples that are not part of the invention are directed to a capillary tube and a force assisted sample ejection mechanism. The capillary tube absorbing and retaining a sample of a first liquid by capillary action when placed in contact with the first liquid. The capillary tube has a generally hollow tubular body that is open on one or both ends. The force assisted sample ejecting mechanism releasing at least part of the sample of the first liquid from the capillary tube when the sample of the first liquid is retained in the capillary tube. The force assisted sample ejecting mechanism releasing the sample of the first liquid by abruptly shaking the capillary tube at least once.

Other herein disclosed concepts that are not part of the invention are further directed to a capillary tube absorbing and retaining a sample of a first liquid by capillary action when placed in contact with the first liquid. The capillary tube has a generally hollow tubular body that is open on one or both ends. The capillary tube being made of a dissolvable polymer that dissolves when submerged in a second liquid.

The concepts disclosed herein are additionally directed to a breakable capillary tube and a force assisted sample ejection mechanism. The breakable capillary tube absorbing and retaining a sample of a first liquid by capillary action when placed in contact with the first liquid. The breakable capillary tube having a generally hollow tubular body that is open on one or both ends. The force assisted sample ejection mechanism releasing at least part of the sample of the first liquid from the breakable capillary tube when the sample of the first liquid is retained in the breakable capillary tube. The force assisted sample ejection mechanism breaking the breakable capillary tube in order to release the first liquid.

### SUMMARY OF THE INVENTION

In accordance with the present invention, there is provided a device as defined in claim 1 and a method as defined in claim 8. In addition, further advantageous embodiments follow from the dependent claims.

It is noted that references to "embodiments" throughout the description which are not under the scope of the appended claims represent possible examples and are therefore not part of the present invention unless the context clearly dictates otherwise. The invention is defined by the appended claims.

### BRIEF DESCRIPTIONS OF THE DRAWINGS

Figure 1 illustrates one example of a sample insertion device and a capillary tube.
Figure 2 illustrates one example of a container.
Figure 3 illustrates one example of a diagnostics device.

### DETAILED DESCRIPTION OF THE INVENTIVE CONCEPT(S)

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. The inventive concepts disclosed herein are capable of other embodiments or of being practiced or carried out in various ways. Also, it is to be understood that the phraseology and terminology employed herein is for the purpose of description and should not be regarded as limiting the inventive concepts disclosed and claimed herein in any way.

In the following detailed description of embodiments of the inventive concepts, numerous specific details are set forth in order to provide a more thorough understanding of the inventive concepts. However, it will be apparent to one of ordinary skill in the art that the inventive concepts within the instant disclosure may be practiced without these specific details. In other instances, well-known features have not been described in detail to avoid unnecessarily complicating the instant disclosure.

As used herein, the terms "comprises," "comprising," "includes," "including," "has," "having" or any other variation thereof, are intended to cover a non-exclusive inclusion. For example, a composition, a process, method, article, or apparatus that comprises a list of elements is not necessarily limited to only those elements but may include other elements not expressly listed or inherently present therein.

As used herein the terms "approximately," "about," "substantially" and variations thereof are intended to include not only the exact value qualified by the term, but to also include some slight deviations therefrom, such as deviations caused by measuring error, manufacturing tolerances, wear and tear on components or structures, settling or precipitation of cells or particles out of suspension or solution, chemical or biological degradation of solutions over time, stress exerted on structures, and combinations thereof, for example.

As used herein, the term "liquid sample" and variations thereof is intended to include, for example, but not limited to, biological fluids, chemical fluids, chemical substances, suspensions, solutions, slurries, mixtures, agglomerations, tinctures, slides, or other preparations of biological fluids, synthetic analogs to biological fluids, and combinations thereof.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present). An inclusive or may be understood as being the equivalent to: at least one of condition A or B.

In addition, use of the "a" or "an" are employed to describe elements and components of the embodiments herein. This is done merely for convenience and to give a general sense of the inventive concepts. This description should be read to include one or at least one and the singular also includes the plural unless it is obvious that it is meant otherwise.

Finally, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

The inventive concepts disclosed herein are generally directed to methods and devices for speeding up the delivery of the delivery of the sample liquid to be tested-housed in a capillary tube-into an assay reaction solution as part of a medical assay. The methods/devices disclosed herein involve, for example, shaking the contents of the capillary tube out into the assay reaction solution, utilizing a capillary tube that dissolves in a second liquid and/or breaking the capillary tube.

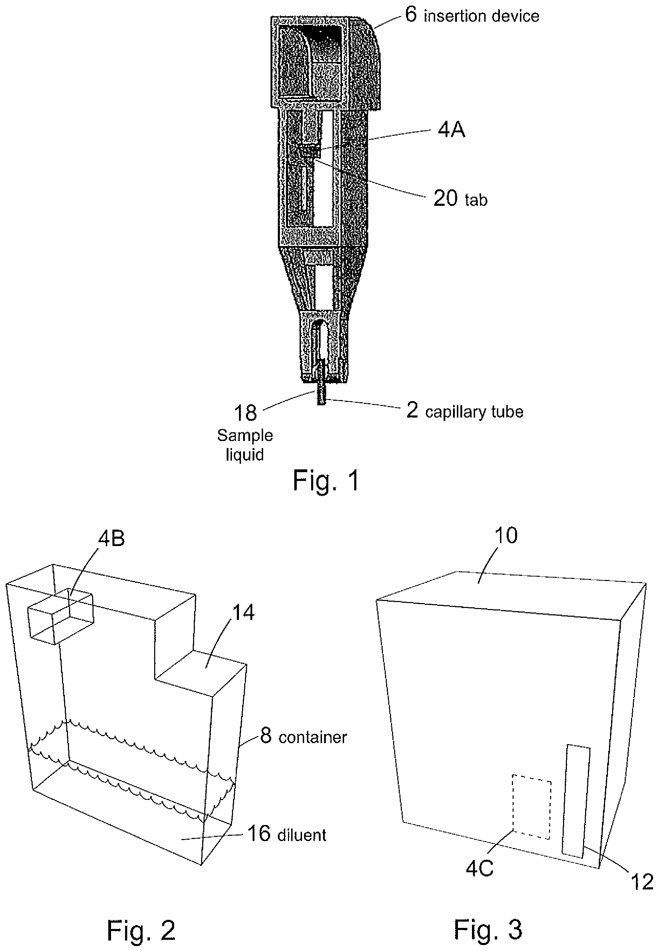

Referring to Figs. 1-3, an exemplary capillary tube 2 is shown. The capillary tube 2 being able to absorb and retain a sample of a sample liquid 18 by capillary action. Sample liquid 18 is any type of liquid. For example, sample liquid 18 is a biological sample such as whole blood, plasma, serum, urine, saliva etc... In the example of whole blood, when placed in contact with the whole blood (such as when the capillary tube 2 is placed against a patient's finger after that finger has been pricked), a portion is drawn into the capillary tube 2by capillary action. The capillary tube 2 is made out of a variety of materials such as, for example but not limited to, glass or plastic. The capillary tube 2 has a generally hollow tubular body that is open on one or both ends. The hollow tubular body of the capillary tube 2 has a cross section resembling a variety of different geometric shapes (for example a triangle, circle, rectangle, pentagon, etc....).

During the medical assay procedure, the sample liquid 18 in capillary tube 2 is combined with a second liquid 16. Second liquid 16 may be an aqueous solution, such as a liquid assay diluent 16. Liquid assay diluent 16 is retained in a container 8. Container 8 of Fig. 2 is intended to function as an example only. It should be appreciated that container 8 takes on a wide variety of shapes and sizes.

In an example, the sample liquid in capillary tube 2 is combined with the liquid assay diluent 16 in the container 8 by forcefully releasing all or at least part of sample liquid 18 into the liquid assay diluent 16 and/or container 8. A forceful release (which also is referred to as splattering) of the sample liquid 18 is accomplished by abruptly shaking the capillary tube 2 at least once via a force assisted sample ejecting mechanism 4. As depicted in Figs. 1-3, one or more, or each, of a insertion device 6 (described in further detail below), the container 8, and a diagnostic device 10 has a force assisted sample ejecting mechanism 4A, 4B, and 4C, respectively. One or more of force assisted sample ejecting mechanisms 4A, 4B, and 4C function in tandem or separately in order to release the sample liquid 18 from the capillary tube 2. Diagnostic Device 10 is configured to receive (via opening 12) the container 8 and interpret the assay results.

The force assisted sample ejecting mechanism 4 operates by one or more of shaking the container 8 one or more times, shaking the capillary tube 2 one or more times, hitting the container 8 one or more times, hitting the capillary tube 2 one or more times, vibrating the container 8 one or more times, vibrating the capillary tube 2 one or more times, or using more force than is needed to place the insertion device 6 in the container 8. Accordingly the force assisted sample ejecting mechanism 4 is located within the container 8 or outside of the container 8. The force assisted sample ejecting mechanism 4 shakes the capillary tube 2 but not the container 8 or the capillary tube 2 and the container 8.

Insertion device 6 permanently or temporarily grips the capillary tube 2 and is used to aid in the insertion of the capillary tube 2 into the container 8. Examples of insertion devices 6 include, for example, tweezers, parallel groves between which the capillary tube 2 can be held in place, forceps, etc. Alternatively, the insertion device 6 is integrated into the container 8 itself. In yet another alternative that is not part of the invention, the insertion device 6 is used to simply drop the capillary tube 2 into the container 8. In the embodiment depicted in Figs. 1-2, the insertion device 6 is inserted through opening 14 in container 8.

In the example depicted in Fig. 1, the capillary tube 2 is affixed to the sample insertion device 6. Once the capillary tube 2 has been loaded with sample liquid 18, the insertion device 6 is inserted into container 8 such that the capillary tube 2 is placed in a test position within the container 8. In the test position, the capillary will be fully or partially submerged with the assay diluent 16 while the assay takes place. In an embodiment, the force assisted sample ejecting mechanism 4 is activated while placing the insertion device 6 in the container 8.

According to another example that is not part of the invention, a method of releasing at least part of sample liquid includes abruptly shaking the capillary tube 2-which retains at least part of the sample liquid 18-at least once. The shaking motion being sufficient to result in the ejection of at least part of the first liquid from the capillary tube 2. When the capillary tube 2 is positioned above or within the container 8, the shaking motion involving: (1) shaking the capillary tube 2 but not a container 8 or (2) the capillary tube 2 and the container 8. The shaking takes place upon inserting the insertion device 6, to which the capillary tube is affixed, into the container 8 or after the insertion device 6 has been inserted. The container 8 retains the assay diluent 16 prior to or after the shaking. For example, Fig. 1 depicts an insertion device 6 with a tab 20. When the insertion device 6 is inserted into container 8, the tab 20 operates to with the housing of container 8 to shake the capillary tube 2 as the tab 20 passes into the housing. The size and rigidity of the tab 20 can be selected in order to optimize the shaking movement imparted onto the capillary tube 2 as the tab 20 engages the container 8.

In another example that is not part of the invention, capillary tube 2 is dissolvable. The dissolvable capillary tube 2 is utilized alone or in combination with the force assisted ejection mechanism 4. Dissolvable capillary tubes 2 are made of a dissolvable material, such as a polymer, wherein the dissoluble polymer dissolves when in contact with the assay diluent 16. Dissolvable capillary tube 2 is affixed to an insertion device 6 and inserted into the container 8 using the insertion device 6 or simply dropped into the container 8.

In order to prevent the capillary tube 2 from dissolving before it comes into contact with the assay diluent 16, the inside of the capillary tube 2 has a coating which prevents the sample liquid 18 from dissolving the capillary tube 2 but does not prevent the sample liquid 18 from doing so. Alternatively, where the sample liquid 18 and the assay diluent 16 have different pH values, the capillary tube 2 is composed of a material that is pH sensitive and dissolves in the assay diluent 16 but not the liquid sample 18. In the example where the sample liquid 18 is whole blood, the capillary tube 2 is comprised of a polylactide which is not dissolved by whole blood (which has a near neutral pH) but in an assay diluent 16 having a higher or lower pH. This would cause the polymer to hydrolyze when the capillary tube 2 is at least partially submerged, thereby dissolving the capillary tube 2.

According to the invention, capillary tube 2 is breakable. The breakable capillary tube 2 may also be dissolvable as disclosed above. When utilizing breakable capillary tubes 2, force assisted ejection mechanism 4 operates to break the breakable capillary tube 2 in order to release the first liquid. Breakable capillary tube 2 is made out of any material that is breakable, such as glass, plastic, etc... Breakable capillary tube 2 is affixed to an insertion device 6 and inserted into the container 8 using the insertion device 6 or simply dropped into the container 8. When using an insertion device 6, the force assisted sample ejection mechanism 4 operates to break the capillary tube 2 during insertion into the container 8 or once the capillary tube 2 is positioned in the test position. Alternatively, capillary tube 2 is dropped into the container 8 in order to break it or broken prior to being inserted into the cartridge.

As described above, the force assisted sample ejection mechanism 4 can be part of the insertion device 6 and/or the container 8 and can be located within the container 8 or outside of the container 8. In order to break a breakable capillary tube 2, the force assisted sample ejection mechanism 4 operates by at least one of breaking, hitting, crushing, shattering, piercing, or snapping the capillary tube 2 in a manner sufficient to break it into at least two pieces.

## Claims

1. A device (10) comprising:
a breakable capillary tube (2) configured to absorb and retain a sample of a first liquid (18) by capillary action when placed in contact with the first liquid (18), the breakable capillary tube (2) having a generally hollow tubular body that is open on one or both ends;
a container (8) able to retain the sample of the first liquid (18) as well as a second liquid (16);
an insertion device (6), the breakable capillary tube (2) being affixed to the insertion device (6);
a force assisted sample ejection mechanism (4), the force assisted sample ejection mechanism (4) configured to
release at least part of the sample of the first liquid (18) from the breakable capillary tube (2) when the sample of the first liquid (18) is retained in the breakable capillary tube (2),
break the breakable capillary tube (2) into at least two pieces in order to release the first liquid (18);
**characterized in that**,
when the insertion device (6) is placed in the container (8) the breakable capillary tube (2) is placed in a test position, wherein the force assisted sample ejection mechanism (4) breaks the breakable capillary tube (2) once the breakable capillary tube (2) is in the test position.

2. The device (10) of claim 1, in which the force assisted sample ejection mechanism (4) is part of the insertion device (6).

3. The device (10) of claim 2, wherein the force assisted sample ejection mechanism (4) is activated while placing the insertion device (6) in the container (8).

4. The device (10) of claim 3, wherein the force assisted sample ejection mechanism (4) is part of the container (8).

5. The device (10) of claim 1, in which the force assisted sample ejection mechanism (4) is located within the container (8).

6. The device (10) of claim 1, in which the force assisted sample ejection mechanism (4) is located outside of the container (8).

7. The device (10) of claim 1, wherein the force assisted sample ejection mechanism (4) operates by at least one of breaking, hitting, crushing, shattering, piercing, or snapping the breakable capillary tube (2).

8. A method of releasing at least part of a first liquid (18) retained in a capillary tube (2) from the capillary tube (2), the method comprising:
breaking a breakable capillary tube (2), the breakable capillary tube (2) absorbing and retaining a sample of a first liquid (18) by capillary action when placed in contact with the first liquid (18), the breakable capillary tube (2) having a generally hollow tubular body that is open on one or both ends;
**characterized in that**,
the breakable capillary tube (2) is affixed to an insertion device (6);
and the breakable capillary tube (2) is broken into at least two pieces by a force assisted sample ejection mechanism (4);
when the insertion device (6) is placed in a container (8) and the breakable capillary tube (2) is in a test position.

## Patentansprüche

1. Vorrichtung (10), umfassend:
ein brechbares Kapillarröhrchen (2), das ausgestaltet ist, um eine Probe einer ersten Flüssigkeit (18) mittels Kapillarwirkung zu absorbieren und festzuhalten, wenn es in Kontakt mit der ersten Flüssigkeit (18) platziert wird, wobei das brechbare Kapillarröhrchen (2) einen allgemein hohlen Röhrenkörper aufweist, der an einem oder beiden Enden offen ist;
einen Behälter (8), der die Probe der ersten Flüssigkeit (18) sowie eine zweite Flüssigkeit (16) festhalten kann;
eine Einführvorrichtung (6), wobei das brechbare Kapillarröhrchen (2) an der Einführvorrichtung (6) angebracht ist;
einen kraftunterstützten Probenauswerfmechanismus (4), wobei der kraftunterstützte Probenauswerfmechanismus (4) ausgestaltet ist zum
Freisetzen mindestens eines Teils der Probe der ersten Flüssigkeit (18) aus dem brechbaren Kapillarröhrchen (2), wenn die Probe der ersten Flüssigkeit (18) in dem brechbaren Kapillarröhrchen (2) festgehalten wird,
Brechen des brechbaren Kapillarröhrchens (2) in mindestens zwei Stücke, um die erste Flüssigkeit (18) freizusetzen;
**dadurch gekennzeichnet, dass**,
wenn die Einführvorrichtung (6) in dem Behälter (8) platziert wird, das brechbare Kapillarröhrchen (2) in einer Testposition platziert wird, wobei der kraftunterstützte Probenauswerfmechanismus (4) das brechbare Kapillarröhrchen (2) bricht, sobald das brechbare Kapillarröhrchen (2) sich in der Testposition befindet.

2. Vorrichtung (10) nach Anspruch 1, wobei der kraftunterstützte Probenauswerfmechanismus (4) Teil der Einführvorrichtung (6) ist.

3. Vorrichtung (10) nach Anspruch 2, wobei der kraftunterstützte Probenauswerfmechanismus (4) aktiviert wird, während die Einführvorrichtung (6) in dem Behälter (8) platziert wird.

4. Vorrichtung (10) nach Anspruch 3, wobei der kraftunterstützte Probenauswerfmechanismus (4) Teil des Behälters (8) ist.

5. Vorrichtung (10) nach Anspruch 1, wobei der kraftunterstützte Probenauswerfmechanismus (4) sich innerhalb des Behälters (8) befindet.

6. Vorrichtung (10) nach Anspruch 1, wobei der kraftunterstützte Probenauswerfmechanismus (4) sich außerhalb des Behälters (8) befindet.

7. Vorrichtung (10) nach Anspruch 1, wobei der kraftunterstützte Probenauswerfmechanismus (4) durch mindestens eines von Brechen, Schlagen, Quetschen, Zersplittern, Durchstechen oder Abknicken des brechbaren Kapillarröhrchens (2) arbeitet.

8. Verfahren zum Freigeben von mindestens einem Teil der ersten Flüssigkeit (18), die in einem Kapillarröhrchen (2) festgehalten wird, aus dem Kapillarröhrchen (2), wobei das Verfahren umfasst:
Brechen eines brechbaren Kapillarröhrchens (2), wobei das brechbare Kapillarröhrchen (2) eine Probe einer ersten Flüssigkeit (18) mittels Kapillarwirkung absorbiert und festhält, wenn es in Kontakt mit der ersten Flüssigkeit (18) platziert wird, wobei das brechbare Kapillarröhrchen (2) einen allgemein hohlen Röhrenkörper aufweist, der an einem oder beiden Enden offen ist;
**dadurch gekennzeichnet, dass**
das brechbare Kapillarröhrchen (2) an einer Einführvorrichtung (6) angebracht ist;
und das brechbare Kapillarröhrchen (2) durch einen kraftunterstützten Probenauswerfmechanismus (4) in mindestens zwei Stücke gebrochen wird;
wenn die Einführvorrichtung (6) in einem Behälter (8) platziert wird und das brechbare Kapillarröhrchen (2) sich in einer Testposition befindet.

## Revendications

1. Dispositif (10) comprenant :
un tube capillaire à rupture (2) configuré pour absorber et retenir un échantillon d'un premier liquide (18) par action capillaire lorsqu'il est placé en contact avec le premier liquide (18), le tube capillaire à rupture (2) ayant un corps tubulaire généralement creux qui est ouvert à une ou aux deux extrémités ;
un récipient (8) capable de retenir l'échantillon du premier liquide (18) ainsi qu'un second liquide (16) ;
un dispositif d'insertion (6), le tube capillaire à rupture (2) étant fixé au dispositif d'insertion (6) ;
un mécanisme d'éjection d'échantillon assisté par force (4), le mécanisme d'éjection d'échantillon assisté par force (4) étant configuré pour
libérer au moins une partie de l'échantillon du premier liquide (18) depuis le tube capillaire à rupture (2) lorsque l'échantillon du premier liquide (18) est retenu dans le tube capillaire à rupture (2),
rompre le tube capillaire à rupture (2) en au moins deux morceaux afin de libérer le premier liquide (18) ;
**caractérisé en ce que**,
lorsque le dispositif d'insertion (6) est placé dans le récipient (8), le tube capillaire à rupture (2) est placé dans une position d'essai, dans lequel le mécanisme d'éjection d'échantillon assisté par force (4) rompt le tube capillaire à rupture (2) une fois que le tube capillaire à rupture (2) est en position d'essai.

2. Dispositif (10) selon la revendication 1, dans lequel le mécanisme d'éjection d'échantillon assisté par force (4) fait partie du dispositif d'insertion (6).

3. Dispositif (10) selon la revendication 2, dans lequel le mécanisme d'éjection d'échantillon assisté par force (4) est activé en plaçant le dispositif d'insertion (6) dans le récipient (8).

4. Dispositif (10) selon la revendication 3, dans lequel le mécanisme d'éjection d'échantillon assisté par force (4) fait partie du récipient (8).

5. Dispositif (10) selon la revendication 1, dans lequel le mécanisme d'éjection d'échantillon assisté par force (4) est situé à l'intérieur du récipient (8).

6. Dispositif (10) selon la revendication 1, dans lequel le mécanisme d'éjection d'échantillon assisté par force (4) est situé à l'extérieur du récipient (8).

7. Dispositif (10) selon la revendication 1, dans lequel le mécanisme d'éjection d'échantillon assisté par force (4) fonctionne par au moins un des moyens suivants : rupture, frappe, écrasement, éclatement, perçage ou claquement du tube capillaire à rupture (2).

8. Procédé pour libérer au moins une partie d'un premier liquide (18) retenu dans un tube capillaire (2) depuis le tube capillaire (2), le procédé comprenant les étapes consistant à :
rompre un tube capillaire à rupture (2), le tube capillaire à rupture (2) absorbant et retenant un échantillon d'un premier liquide (18) par action capillaire lorsqu'il est placé en contact avec le premier liquide (18), le tube capillaire à rupture (2) ayant un corps tubulaire généralement creux qui est ouvert à une ou aux deux extrémités ;
**caractérisé en ce que**,
le tube capillaire à rupture (2) est fixé à un dispositif d'insertion (6) ;
et le tube capillaire à rupture (2) est rompu en au moins deux morceaux par un mécanisme d'éjection d'échantillon assisté par force (4) ;
lorsque le dispositif d'insertion (6) est placé dans un récipient (8) et que le tube capillaire à rupture (2) est en position d'essai.
